# EUROPEAN PATENT SPECIFICATION

(11) **EP 3 698 759 B1**
(45) Date of publication and mention of the grant of the patent: **08.05.2024**
(21) Application number: 18869140.6
(22) Date of filing: 17.10.2018
(51) Int. Cl.: A61F 2/24, A61B 17/00, A61B 17/04, A61M 25/00

(54) **VALVE LEAFLET REPAIR SYSTEM FOR USE IN PERFORMING MINIMALLY INVASIVE SURGERY**
KLAPPENSEGELREPARATURSYSTEM ZUR VERWENDUNG BEI DER DURCHFÜHRUNG MINIMALINVASIVER CHIRURGIE
SYSTÈME DE RÉPARATION DE FEUILLET DE VALVULE DESTINÉ À ÊTRE UTILISÉ DANS LA RÉALISATION D'UNE CHIRURGIE MINI-INVASIVE

(30) Priority: 20.10.2017 CN 201710985383; 03.05.2018 CN 201810414365
(43) Date of publication of application: 26.08.2020
(73) Proprietor: Beijing Med-Zenith Medical Scientific Co., Ltd., Beijing 101312 (CN)
(72) Inventor: DAI, Gaoxu, Beijing 101312 (CN); LI, Yang, Beijing 101312 (CN); YANG, Yuting, Beijing 101312 (CN); ZHOU, Qingliang, Beijing 101312 (CN); KE, Danian, Beijing 101312 (CN); MENG, Jian, Beijing 101312 (CN)
(74) Representative: McKinnon, Alistair James
(86) International application number: PCT/CN2018/110684
(87) International publication number: WO 2019/076325

(56) References cited:
- EP-A2- 2 626 014
- EP-A2- 2 626 014
- WO-A1-2013/003228
- WO-A1-2016/192481
- WO-A1-2017/059426
- CN-A- 101 257 852
- CN-A- 104 873 307
- CN-A- 106 999 174
- CN-A- 107 822 743
- US-A1- 2007 032 796
- US-A1- 2017 258 588

## Description

### Cross-Reference to Related Application

The present disclosure claims priority on the basis of Chinese patent application no. 2017109853836, which is filed to SIPO on 20 October 2017 and entitled "SYSTEM FOR REPAIRING VALVE LEAFLETS IN MINIMALLY INVASIVE SURGERY", and Chinese patent application no. 2018104143657, which is filed to SIPO on 3 May 2018 and entitled "SYSTEM FOR REPAIRING VALVE LEAFLETS IN MINIMALLY INVASIVE SURGERY".

### Technical Field

The present disclosure relates to the technical field of medical instruments, and in particular, to a system for repairing valve leaflets in minimally invasive surgery.

### Background

Minimally invasive surgery causing a smaller wound to a human body becomes a more and more widely used medical technique.Many minimally invasive implanted instruments can only be implanted by performing complex steps by means of an operating handle, and how to make a it easier and more convenient to perform these steps is a development direction of minimally invasive instruments.

A normal valve can ensure blood to flow smoothly in one direction, and effectively pump the blood out of a chamber without regurgitation. Many diseases, such as the rheumatic heart disease and endocarditis, bring about valvular lesions, causing damage to the function of valve, and stenosis and regurgitation are common valvular diseases. The stenosis is caused by the inability of the valve to open completely, resulting in the blockage in blood flow, and thickening of valve leaflets caused by calcification is a common cause of the stenosis. The regurgitation is caused by the inability of the valve leaflet to close completely, resulting in the backflow of blood to the blood pumping chamber, and valve ring expanding, valve leaflet prolapse and valve leaflet movement blockage are main causes of the regurgitation.

The mitral valve and the tricuspid valve are composed of leaflets attached to the valve rings, these leaflets are supported at free edges thereof by means of chordae tendineae, and the chordae tendineae are attached to an inner wall of the ventricle and the papillary muscles. However, sometimes, in the case that one or more chordae tendineae are loose or ruptured, the valve prolapse occurs, and therefore the seal normally provided between the atrium and the ventricle is damaged, resulting in blood backflow to the atrium during systole.

However, in the related technique, the implantation in valve leaflet repair surgery is difficult to operate, and the surgery intensity and difficulty for the surgeon are relatively high.

WO 2017/059426 describes an apparatus including a handle, an actuator, a pusher device, and a puncture member. A distal anchor is disposed at a distal end portion of an artificial chordae and is in a delivery configuration. The artificial chordae is coupled to the actuator and extends through a lumen of the puncture member. The actuator can be actuated to move the puncture member distally a preset distance, and to move the pusher device distally such that at least a portion of the distal anchor is moved distal to the distal end of the puncture member and the distal anchor is moved from its delivery configuration to a deployed configuration.

WO 2016/192481 describes a heart valve repair device comprising: a sealed sheath; a catcher located at a distal end of the sealed sheath for catching and fixing a heart valve cusp; a puncture needle located in the sealed sheath for puncturing the heart valve cusp caught by the catcher; an artificial chordae tendineae capable of puncturing into the heart valve along with the puncture needle and being for repairing the heart valve; and a handle located at a proximal end of the sealed sheath and used to operate the heart valve repair device.

### Summary

In view of this, the present disclosure provides a system for repairing valve leaflets in minimally invasive surgery, which is a system enabling the surgery to be completed on a beating heart and accurately repairing the function of the valve leaflet. The operation is easy, and release and repair operations can be realized by one step, reducing operation difficulty and patient pain in the surgery.

The invention is disclosed in the appended claims.

The technical solution used in the present disclosure is:
A system for repairing valve leaflets in minimally invasive surgery, including: a delivery device and a restoration component, wherein the delivery device can perform a linkage release operation; the delivery device includes an operating handle, a positioning tube, an outer tube clamp, an outer delivery tube, a delivery needle and
a pushing tube; and the restoration component includes an anchor and a connection wire.

Further, the operating handle includes a pull rod, a push member, a handle, a release button, a sliding member, and a safety button; the pull rod is connected to the pushing tube, and the push member is fitted outside the pull rod; the push member is connected to the delivery needle, and the handle is fitted outside the push member; the handle is connected to the outer delivery tube; the pull rod and the push member are provided with ejection mechanisms, and the pull rod and the push member are cooperatively driven by means of a spring; and the positioning tube is fitted outside the outer delivery tube, the outer delivery tube is fitted outside the delivery needle, the pushing tube is located inside the delivery needle, and the pushing tube is used for releasing the restoration component inside the delivery needle.

Further, the pull rod is provided with an evacuation connector and a tubular structure capable of stopping bleeding and being cut off to disconnect the connection wire, and a spring mechanism is provided between an outer side of the pull rod and an inner side of the push member.

Further, a snapping member is provided on the inner side of the push member, and is used for controlling an ejection of the pull rod, and a spring mechanism is provided between an outer side of the push member and an inner side of the handle.

Further, the sliding member is provided on the handle, a release trigger part is located in the push member, and pulling out the pull handle enables the sliding member to slide to a release position, so as to unlock the snapping member and release the pull rod.

Further, the release button is provided on the handle, the push member is ejected after the release button is triggered, and the sliding member can trigger the snapping member after the push member is ejected, so as to trigger an ejection of the pull rod. Further, the handle is provided with a safety button for preventing misoperation, and the safety button is used for preventing misoperation from triggering the release button.

Further, the positioning tube fits with the outer tube clamp, such that the distal end of the positioning tube is flush with the distal end of the outer delivery tube, so as to realize intraoperative stopping of bleeding and protection of the valve leaflet at the implantation position.

Further, the anchor is made of a metal or polymer material, and the anchor is anchored to the valve leaflet.

Further, the connection wire is connected to the anchor, and the connection wire is made of polymer material.

In one embodiment of the present disclosure, the restoration component is connected to the delivery device; the operating handle has a three-layered internal structure, including the pushing tube, the delivery needle and the outer delivery tube from inside to outside; the operating handle includes a pull rod, a push member and a handle; the push member is fitted outside the pull rod, and the handle is fitted outside the push member; the operating handle is provided with a first tube seat, a second tube seat and a third tube seat in sequence in an extension direction from left to right; the pull rod is connected to the first tube seat, the push member is connected to the second tube seat, and the handle is connected to the third tube seat; one end of the pushing tube is connected to the first tube seat, and the other end of the pushing tube passes through the center of the second tube seat; one end of the delivery needle is connected to the second tube seat, and the other end of the delivery needle passes through the center of the third tube seat; one end of the outer delivery tube is connected to the third tube seat; the other end of the outer delivery tube passes through the centers of the outer tube clamp and the positioning tube; and the anchor is connected to the connection wire.

Further, the first tube seat is provided with a first engaging groove, the pushing tube is provided with a first engaging piece, and the first engaging groove fits with the first engaging piece;
The second tube seat is provided with a second engaging groove, the delivery needle is provided with a second engaging piece, and the second engaging groove fits with the second engaging piece;
And the third tube seat is provided with a third engaging groove, the outer delivery tube is provided with a third engaging piece, and the third engaging groove fits with the third engaging piece.

Further, a first spring mechanism is provided between an outer side of the pull rod and an inner side of the push member, and the pull rod is drivingly connected to the push member by means of the first spring mechanism;
And a second spring mechanism is provided between an outer side of the push member and an inner side of the handle, and the push member is drivingly connected to the handle by means of the second spring mechanism.

Further, the pull rod is provided with an evacuation connector, a stretching device, a tubular structure, and a first slider which can slide in the push member, and the tubular structure can stop bleeding and, when being cut off, enables the connection wire to be disconnected, so as to make the anchor disengage from the delivery device;
And the stretching device includes a wire clip and a first spring, and the wire clip is connected to the connection wire.

Further, the push member is internally provided with a first sliding groove allowing the pull rod to slide, and the first sliding groove is adapted to the first slider; and a second slider which can slide within the handle is provided outside the push member. Further, the push member is provided with a square structure, a snapping member is provided in the square structure, one end of the snapping member is provided with a second spring, and the snapping member locks the pull rod under the action of the second spring.

Further, the handle is provided with a sliding member and a second sliding groove allowing the push member to slide, and the second sliding groove is adapted to the second slider; and the sliding member is located at a tail end of the handle, the sliding member can slide to the release position after the push member is pulled out, and the sliding member is used for unlocking the snapping member when the push member is ejected, so as to trigger an ejection of the pull rod.

Further, a release button is provided at a front end of the handle, a third spring is provided at a lower end of the release button, after the push member is pulled out, the release button can lock the push member under the action of the third spring, and the push member is ejected after the release button is triggered.

Further, a safety button is further provided on the handle, the safety button is provided adjacent to the release button, a fourth spring is provided at a tail end of the safety button, and the safety button remains locked by means of a force of the fourth spring.

Further, a safety button reset hole is further provided on the handle, the safety button reset hole is provided adjacent to the safety button, and the safety button reset hole is used for resetting the safety button after the safety button is unlocked.

Compared with the prior art, the beneficial effects achieved in the present disclosure lies in that the system for repairing valve leaflets in minimally invasive surgery in the present disclosure includes a delivery device and a restoration component, and the delivery device and the restoration component can perform a linkage release operation; the delivery device includes an operating handle, a positioning tube, an outer tube clamp, an outer delivery tube, a delivery needle and a pushing tube; and the restoration component includes an anchor and a connection wire. The system provided in the present disclosure enables the surgery to be completed on a beating heart; the surgery can accurately repair the function of the valve leaflets by means of the navigation function of modern imaging apparatus, such as ultrasonic apparatus, thoracoscopic apparatus and laparoscopic apparatus; and the operation is easy, release and repair operations can be realized by means of one step, reducing operation difficulty and patient pain in the surgery, improving the successful rate of the surgery.

### Brief Description of the Drawings

Fig. 1 is a sectional view of an operating handle provided in the present disclosure;
Fig. 2 is a schematic structural diagram of a non-operating handle portion of a delivery device provided in the present disclosure;
Fig. 3 is a schematic configuration diagram of a restoration component of a valve leaflet repair system provided in the present disclosure;
Fig. 4 is exploded views of a first tube seat and a pushing tube provided in the present disclosure;
Fig. 5 is exploded views of a second tube seat and a delivery needle provided in the present disclosure;
Fig. 6 is exploded views of a third tube seat and an outer delivery tube provided in the present disclosure;
Fig. 7 is a schematic diagram of the cooperation between a stretching device and a tubular structure provided in the present disclosure;
Fig. 8 is a diagram of the position of a first sliding groove provided in the present disclosure;
Fig. 9 is a diagram of the position of a second sliding groove provided in the present disclosure;
Fig. 10 is a diagram of the position of a second slider provided in the present disclosure;
Fig. 11 is a schematic structural diagram of a front end of an operating handle provided in the present disclosure; and
Fig. 12 is a perspective view of the structure of an operating handle provided in the present disclosure. The meaning represented by the reference signs is as follows:
   1-Pull rod; 2-Push member; 3-Handle; 4-Tubular structure; 5-Snapping member; 6-Sliding member; 7-Release button; 8-Safety button; 9-Outer delivery tube; 10-Delivery needle; 11-Pushing tube; 12-First spring mechanism; 13-Second spring mechanism; 14-First tube seat; 15-Second tube seat; 16-Third tube seat; 17-Evacuation connector; 18-First slider; 19-Positioning tube; 20-Outer tube clamp; 21-Restoration component; 22-First engaging groove; 23-Second engaging groove; 24-Third engaging groove; 25-First engaging piece; 26-Second engaging piece; 27-Third engaging piece; 28-Wire clip; 29-Spring; 30-First sliding groove; 31-Second sliding groove; 32-Second slider; 33-Safety button reset hole.

### Detailed Description of the Embodiments

Hereinafter, specific embodiments of the present disclosure will be described in detail with reference to the accompanying drawings.

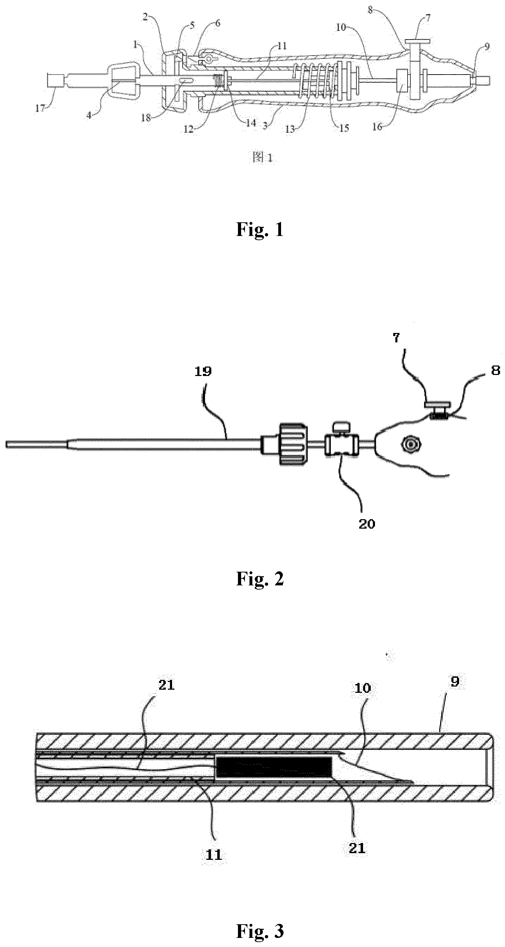

Referring to Figs. 1 and 3, Fig. 1 is a schematic structural diagram of an operating handle provided in the present disclosure, and Fig. 3 is a schematic configuration diagram of a restoration component of a valve leaflet repair system provided in the present disclosure. The present disclosure provides a system for repairing valve leaflets in minimally invasive surgery, including a delivery device and a restoration component 21, the restoration component 21 is connected to the delivery device; and the restoration component 21 includes an anchor and a connection wire, and the anchor is connected to the connection wire. The connection wire of the restoration component 21 is connected to a wire clip 28 of a pull rod 1 through a pushing tube 11, and the anchor is provided at an end of the pushing tube 11 and positioned inside the delivery needle 10. The anchor is made of a metal or polymer material, and the anchor is anchored to the valve leaflet; the connection wire is made of polymer material, such as expanded polytetrafluoroethylene or polyester material.

The delivery device includes an operating handle, a positioning tube 19, an outer tube clamp 20, an outer delivery tube 9, a delivery needle 10 and a pushing tube 11; and in an embodiment of the present disclosure, the operating handle includes the pull rod 1, a push member 2, a handle 3, a release button 7, a sliding member 6 and a safety button 8. The push member 2 is fitted outside the pull rod 1; the handle 3 is fitted outside the push member 2; a first spring mechanism 12 is fitted on an outer side of the pull rod 1 and on the inner side of the push member 2, and the pull rod 1 and the push member 2 are able to be cooperatively driven by means of a spring of the first spring mechanism 12, so that the pull rod 1 axially moves relative to the push member 2; and a second spring mechanism 13 is fitted on an outer side of the push member 2, and the push member 2 and the handle 3 are able to be cooperatively driven by means of the second spring mechanism 13, so that the push member 2 axially moves relative to the handle 3.

A snapping member 5 is provided in the push member 2, the snapping member 5 restricts an ejection of the pull rod 1 by means of the spring mechanisms, the push member 2 is internally provided with a sliding groove allowing the pull rod 1 to slide and the first spring mechanism 12 capable of accommodating the pull rod 1 and ejecting the pull rod 1, and a slider capable of sliding in the handle 3 is provided outside the push member 2. The handle 3 is provided with the sliding member 6, and after the handle 3 is pulled out, the sliding member 6 automatically slides to a position triggering the snapping member; the handle 3 is provided with the release button 7, and after the release button 7 is pressed, the push member 2 is ejected forward under the action of the second spring mechanism 13; and the handle 3 is internally provided with a limiting slot allowing the push member 2 to slide, and when the push member 2 is ejected forward under the action of the second spring mechanism 13, the sliding member 6 triggers the snapping member 5, so that the pull rod 1 is ejected forward under the action of the first spring mechanism 12. The handle 3 is provided with the safety button 8 for controlling the release button 7 to prevent misoperations from triggering the release button 7.

The operating handle has a three-layered internal structure, including the pushing tube 11, the delivery needle 10 and the outer delivery tube 9 from inside to outside; the positioning tube 19 (see figure 2) is fitted outside the outer delivery tube 9, the outer delivery tube 9 is fitted outside the delivery needle 10, and the pushing tube 11 is provided inside the delivery needle 10 for releasing the restoration component; the operating handle is provided with a first tube seat 14, a second tube seat 15 and a third tube seat 16 in sequence in an extension direction from left to right (with respect to the reference orientation of Fig. 1); the pull rod 1 is connected to the first tube seat 14, and the push member 2 is connected to the second tube seat 15; one end of the pushing tube 11 is connected to the first tube seat 14, and the other end of the pushing tube passes through the center of the second tube seat 15; one end of the delivery needle 10 is connected to the second tube seat 15, and the other end of the delivery needle passes through the center of the third tube seat 16; one end of the outer delivery tube 9 is connected to the third tube seat 16; and the other end of the outer delivery tube passes through the center of the outer tube clamp 20 and the positioning tube 19.

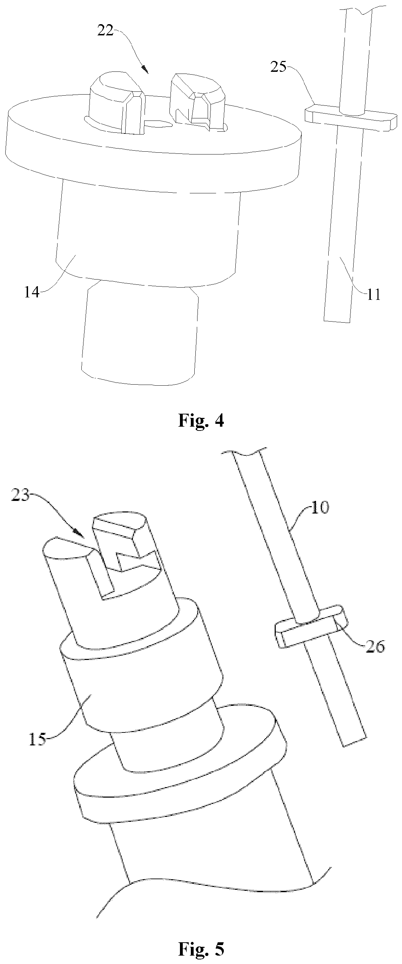

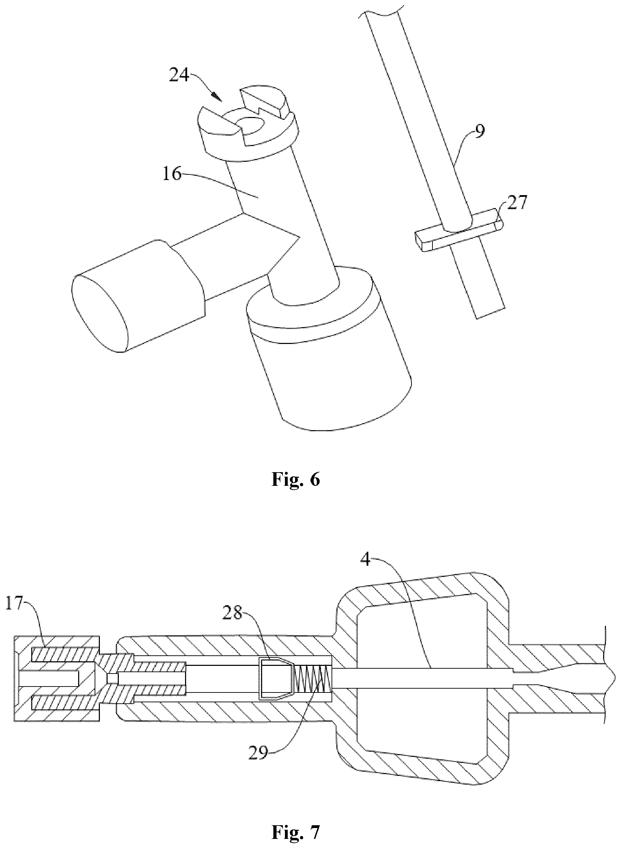

Fig. 4 is exploded views of the first tube seat and the pushing tube provided in the present disclosure; Fig. 5 is exploded views of the second tube seat and the delivery needle provided in the present disclosure; and Fig. 6 is exploded views of the third tube seat and the outer delivery tube provided in the present disclosure. Referring to Figs. 4 to 6, further, the first tube seat 14 is provided with a first engaging groove 22, the pushing tube 11 is provided with a first engaging piece 25, and the first engaging groove 22 fits with the first engaging piece 25; a part of the pushing tube 11 below the first engaging piece 25 is adhered to the first tube seat 14 by gluing; the second tube seat 15 is provided with a second engaging groove 23, the delivery needle 10 is provided with a second engaging piece 26, and a part of the delivery needle 10 below the second engaging piece 26 is adhered to the second tube seat 15 by gluing; the second engaging groove 23 fits with the second engaging piece 26; the third tube seat 16 is provided with a third engaging groove 24, the outer delivery tube 9 is provided with a third engaging piece 27, a part of the outer delivery tube 9 below the third engaging piece 27 is adhered to the third tube seat 16 by gluing, and the third engaging groove 24 fits with the third engaging piece 27; and glue can seal the tubes and the tube seats, prevent the tubes from rotating and prevent the tubes from disengaging from the tube seats, and the fitting between the engaging pieces and the engaging grooves can prevent the tubes from displacing and disengaging from the tube seats due to impact force during ejection. It should be understood that, in other embodiments of the present disclosure, the pushing tube 11, the delivery needle 10 and the outer delivery tube 9 to the respective tube seats may use fastener connections instead of the connection manner of fitting and adhering between engaging pieces and engaging grooves.

Fig. 7 is a schematic diagram of the cooperation between the stretching device and the tubular structure provided in the present disclosure. Referring to Fig. 7, the pull rod 1 is provided with an evacuation connector 17, the stretching device, the tubular structure 4, and a first slider 18 (see Fig. 1) which can slide in the push member 2, and the tubular structure 4 can stop bleeding and, when being cut off, enables the connection wire to be disconnected; and the disengagement of the anchor from the delivery device can be realized after the tubular structure 4 and the connection wire of the restoration component 21 are cut off. A port of the stretching device is provided with the evacuation connector 17; and the stretching device includes the wire clip 28 and a spring 29, and the wire clip 28 is connected to the connection wire of the restoration component 21.

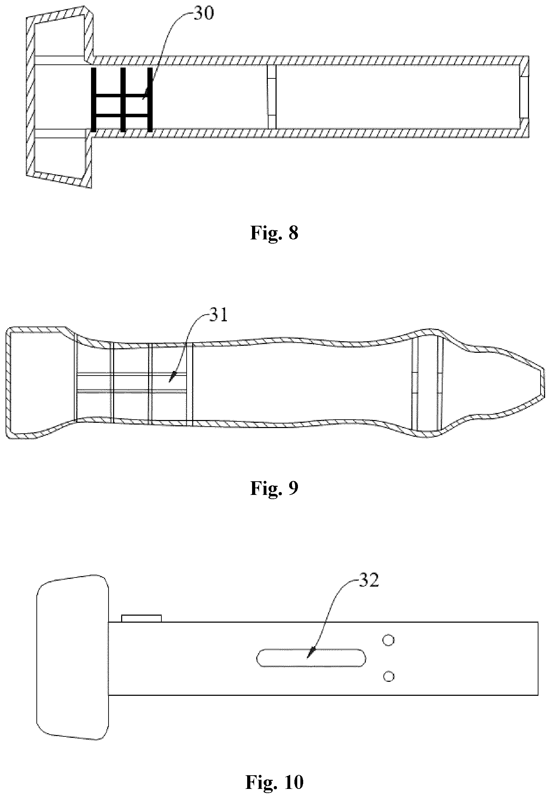

Fig. 8 is a diagram of the position of the first sliding groove provided in the present disclosure; Fig. 9 is a diagram of the position of the second sliding groove provided in the present disclosure; and Fig. 10 is a diagram of the position of the second slider provided in the present disclosure. Referring to Figs. 8 to 10, further, the first spring mechanism 12 is used for ejecting the pull rod 1, the push member 2 is further internally provided with the first sliding groove 30 allowing the pull rod 1 to slide, and the first sliding groove 30 is adapted to the first slider 18 on the pull rod 1; and the handle 3 is internally provided with the second sliding groove 31 allowing the push member 2 to slide, and the second slider 32 is adapted to the second sliding groove 31 on the handle 3.

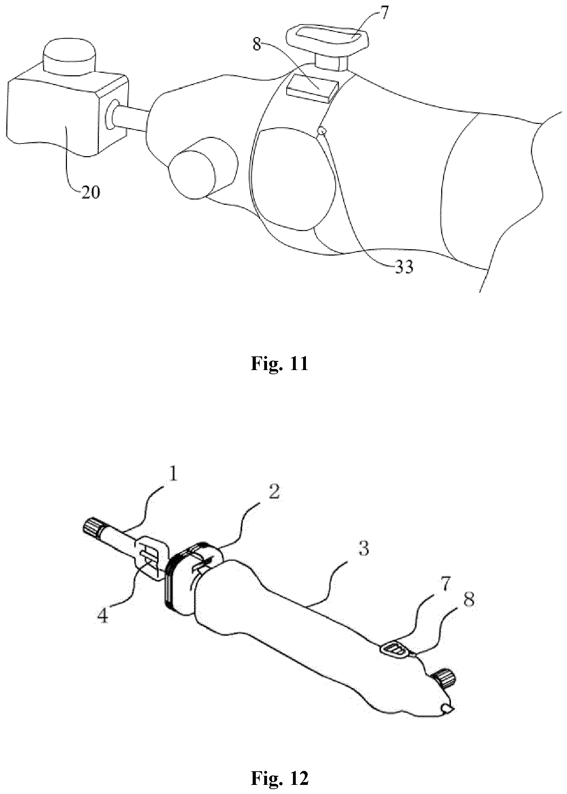

Fig. 11 is a schematic structural diagram of the front end of the operating handle provided in the present disclosure; and Fig. 12 is a perspective view of the structure of the operating handle provided in the present disclosure. Referring to Figs. 11 and 12, a square structure is provided on the left side of the push member 2, the snapping member 5 is provided in the square structure, the snapping member 5 can move up and down along a sliding groove in the square structure, a second spring (not shown) is provided below the snapping member 5, and the snapping member 5 locks the pull rod 1 under the action of the second spring.

The release button 7 is located at the front end (i.e. the right end shown in Fig. 1) of the handle 3, and moves up and down by means of a sliding groove inside the front end of the handle 3, the lower end of the release button 7 is provided with a third spring (not shown), and the release button 7 locks the push member 2 under the action of the third spring.

In the embodiment of the present disclosure, the handle 3 is provided with the second sliding groove 31 allowing the push member 2 to slide, and the second sliding groove 31 is adapted to the second slider 32. The sliding member 6 is located at the tail part (i.e. the left end shown in Fig. 1) of the handle 3, and the sliding member 6 moves by means of a sliding groove and a guide post at the tail part of handle 3 and under the action of a spring. After the push member 2 is pulled out, the sliding member 6 can automatically slide to a release position, and is used for unlocking the snapping member 5 when the push member 2 is ejected, so as to trigger the pull rod 1, and the pull rod 1 is ejected forward under the action of the first spring mechanism 12.

A safety button reset hole 33 is further provided on the handle 3; in an embodiment of the present disclosure, the safety button 8 is provided adjacent to the release button 7, the safety button reset hole 33 is provided adjacent to the safety button 8, and the safety button 8 slides by means of a sliding groove inside the front end of the handle 3; the safety button 8 is used for preventing misoperations on the release button 7, resulting in unexpected release of the restoration component 14; and the safety button reset hole 33 is used for resetting the safety button 8 after the safety button is unlocked.

In the embodiment of the present disclosure, the positioning tube 19 can fit with the outer tube clamp 20, so that the distal end of the positioning tube 19 is parallel to the distal end of the outer delivery tube 9, so as to realize intraoperative stopping of bleeding and protection of the valve leaflets at implantation site. The system for repairing valve leaflets in minimally invasive surgery provided in the present disclosure can be operated easily, and can realize release and repair operations by means of one step, reducing operation difficulty and patient pain in the surgery. The surgery can be completed on a beating heart, and the surgery can accurately repair the function of the valve leaflet by means of the navigation function of modern imaging apparatus, such as ultrasonic apparatus, thoracoscopic apparatus and laparoscopic apparatus. Furthermore, the safety button provided in the present disclosure can prevent unexpected button triggering caused by misoperations. After the protection of the safety button is released, when the handle operation needs to be interrupted in the event of an emergency, the safety button can return to the protection state again by means of the safety button reset hole. The stretching device provided in the operating handle can automatically stretch the artificial chordae into a T-shaped structure after the artificial chordae is released, avoiding the artificial chordae remaining linear-shaped and easily disengaging from the valve leaflet after ejection. The operating handle provided in the embodiment of the present disclosure is composed of a three-layered structure, each layer of which is composed of two parts, i.e. a tube and a tube seat, each of the tube has an engaging piece structure, and each of the tube seat has an engaging groove structure. The lower part of the tube below the engaging piece structure is adhered to the tube seat by gluing, and the engaging piece structure of the tube fits with the engaging groove structure of the tube seat. The advantages of such a structure lies in that: glue can seal the tubes and the tube seats, prevent the tubes from rotating and prevent the tubes from disengaging from the tube seats, and the fitting between the engaging pieces and the engaging grooves can prevent the tubes from displacing and disengaging from the tube seats due to impact forces during ejection.

### Industrial Applicability

The valve leaflet repair system of the present disclosure is suitable for repairing of the valve leaflet in minimally invasive surgery, the system is easy to operate and can finish release and repair operations by one step, and at the same time can reduce operation difficulty, intensity and patient pain in the surgery, improving the successful rate of the surgery.

## Claims

1. A system for repairing valve leaflets in minimally invasive surgery, comprising:
a delivery device and a restoration component (21), wherein the delivery device is configured to perform a release operation of the restoration component;
the delivery device comprises an operating handle, an outer tube clamp (20), an outer delivery tube (9), a delivery needle (10) and a pushing tube (11); wherein the operating handle comprises a pull rod (1), a push member (2), a handle (3), a release button (7), a sliding member (6) and a safety button (8);
wherein the pull rod is connected to the pushing tube (11), and the push member is fitted outside the pull rod; the push member is connected to the delivery needle (10), and the handle is fitted outside the push member; and
wherein the handle is connected to the outer delivery tube (9);
the pull rod and the push member are provided with ejection mechanisms, and the pull rod and the push member are cooperatively driven by means of spring; and the outer delivery tube is fitted outside the delivery needle, the pushing tube is located inside the delivery needle, and the pushing tube is configured to release the restoration component (21) inside the delivery needle;
wherein the restoration component comprises an anchor and a connection wire;
**characterised in that**
the delivery device comprises further a positioning tube (19),
wherein the positioning tube is fitted outside the outer delivery tube and the outer tube clamp is configured to clamp the positioning tube and the outer delivery tube, such that a distal end of the positioning tube is parallel to a distal end of the outer delivery tube.

2. The system for repairing the valve leaflets in minimally invasive surgery as claimed in claim 1, wherein the pull rod (1) is provided with an evacuation connector (17) and a tubular structure (4) capable of stopping bleeding and being cut off to disconnect the connection wire, and a spring mechanism is provided between an outer side of the pull rod and an inner side of the push member.

3. The system for repairing the valve leaflets in minimally invasive surgery as claimed in claim 1, wherein a snapping member (5) is provided on the inner side of the push member (2), and is configured to control an ejection of the pull rod (1), and a spring mechanism is provided between an outer side of the push member and an inner side of the handle (3).

4. The system for repairing the valve leaflets in minimally invasive surgery as claimed in claim 3, wherein the sliding member (6) is provided on the handle, a release trigger part is located in the push member, wherein the pull handle (2) is arranged to enable the sliding member to slide to a release position responsive to the pull handle moving in a direction away from the handle (3), so as to unlock the snapping member and release the pull rod (1).

5. The system for repairing the valve leaflets in minimally invasive surgery as claimed in claim 3, wherein the release button (7) is provided on the handle, the push member is ejected after the release button is triggered, and the sliding member (6) can trigger the snapping member (5) after the push member is ejected, the snapping member configured to unlock the ejection mechanism of the pull rod (1) responsive to being unlocked.

6. The system for repairing the valve leaflets in minimally invasive surgery as claimed in claim 1, wherein the handle (3) is provided with a safety button (8) for preventing misoperations, and the safety button is configured to prevent the misoperations from triggering the release button (7).

7. The system for repairing the valve leaflets in minimally invasive surgery as claimed in any one of claims 1-6, wherein the anchor is made of a metal or polymer material, and the anchor is anchored to the valve leaflet.

8. The system for repairing the valve leaflets in minimally invasive surgery as claimed in any one of claims 1-6, wherein the connection wire is connected to the anchor, and the connection wire is made of a polymer material.

9. The system for repairing the valve leaflets in minimally invasive surgery as claimed in claim 1, wherein the restoration component (21) is connected to the delivery device; the operating handle is provided with a three-layered internal structure, comprising the pushing tube (11), the delivery needle (10) and the outer delivery tube (9) from inside to outside;
the operating handle is provided with a first tube seat (14), a second tube seat (15) and a third tube seat (16) in sequence in an extension direction away from the evacuation connector and toward the outer delivery tube; the pull rod is connected to the first tube seat, the push member is connected to the second tube seat, and the handle is connected to the third tube seat;
one end of the pushing tube is connected to the first tube seat, and the other end of the pushing tube passes through the center of the second tube seat;
one end of the delivery needle is connected to the second tube seat, and the other end of the delivery needle passes through the center of the third tube seat;
one end of the outer delivery tube is connected to the third tube seat; the other end of the outer delivery tube passes through the centers of the outer tube clamp and the positioning tube; and
the anchor is connected to the connection wire.

10. The system for repairing the valve leaflets in minimally invasive surgery as claimed in claim 9,
wherein the first tube seat (14) is provided with a first engaging groove (22), the pushing tube (11) is provided with a first engaging piece (25), and the first engaging groove (22) fits with the first engaging piece (25);
the second tube seat (15) is provided with a second engaging groove (23), the delivery needle (10) is provided with a second engaging piece (26), and the second engaging groove fits with the second engaging piece; and
the third tube seat (16) is provided with a third engaging groove, the outer delivery tube is provided with a third engaging piece (27), and the third engaging groove fits with the third engaging piece.

11. The system for repairing the valve leaflets in minimally invasive surgery as claimed in claim 9, wherein
a first spring mechanism is provided between an outer side of the pull rod (1) and an inner side of the push member (2), and the pull rod is drivingly connected to the push member by means of the first spring mechanism; and
a second spring mechanism is provided between an outer side of the push member and an inner side of the handle (3), and the push member is drivingly connected to the handle by means of the second spring mechanism.

12. The system for repairing the valve leaflets in minimally invasive surgery as claimed in claim 9, wherein the pull rod (1) is provided with an evacuation connector (17), a stretching device, a tubular structure (4), and a first slider (18) arranged to slide in the push member (2), and the tubular structure shaped to stop bleeding and operable to be disconnected from the connection wire, thereby disengaging the anchor from the delivery device; and
the stretching device comprises a wire clip (28) and a first spring, and the wire clip is connected to the connection wire;
and optionally,
wherein the push member is internally provided with a first sliding groove (30) allowing the pull rod to slide, and the first sliding groove is adapted to the first slider; and a second slider (32) arranged to slide within the handle (3) is provided outside the push member;
and further optionally,
wherein the push member is provided with a square structure, a snapping member (5) is provided in the square structure, one end of the snapping member is provided with a second spring, and the snapping member is operable to lock the pull rod under the control of the second spring;
and yet further optionally,
wherein the handle is provided with a sliding member and a second sliding groove (31) allowing the push member to slide, and the second sliding groove is adapted to the second slider; and the sliding member is located at a tail part of the handle, the sliding member arranged to slide to a release position after the push member is pulled out, and the sliding member is operable to unlock the snapping member when the push member is ejected, the snapping member configured to unlock the ejection mechanism of the pull rod responsive to being unlocked.

13. The system for repairing the valve leaflets in minimally invasive surgery as claimed in claim 9,
wherein a release button (7) is provided at a front end of the handle (3), a third spring is provided at a lower end of the release button, after the push member (2) is pulled out, the release button is configured to lock the push member under the action of the third spring, and the push member is ejected after the release button is triggered;
and optionally,
wherein a safety button (8) is further provided on the handle, the safety button is provided adjacent to the release button (7), a fourth spring is provided at a tail part of the safety button, and the safety button is configured to remain locked by means of a force of the fourth spring;
and further optionally,
wherein a safety button reset hole (33) is further provided on the handle, the safety button reset hole is provided adjacent to the safety button, and the safety button reset hole is operable to reset the safety button after the safety button is unlocked.

## Patentansprüche

1. System zum Reparieren von Klappensegeln durch minimalinvasive Chirurgie, umfassend:
eine Zuführvorrichtung und eine Repositionskomponente (21), wobei die Zuführvorrichtung dazu konfiguriert ist, einen Freigabevorgang der Repositionskomponente auszuführen;
wobei die Zuführvorrichtung einen Bediengriff, eine äußere Röhrenklemme (20), eine äußere Zuführröhre (9), eine Zuführnadel (10) und eine Schieberöhre (11) umfasst; wobei der Bediengriff einen Zugstab (1), ein Schubelement (2), einen Griff (3), eine Freigabetaste (7), ein Gleitelement (6) und eine Sicherheitstaste (8) umfasst; wobei der Zugstab mit der Schieberöhre (11) verbunden ist und das Schubelement außerhalb des Zugstabs montiert ist; wobei das Schubelement mit der Zuführnadel (10) verbunden ist und der Griff außerhalb des Schubelements montiert ist; und
wobei der Griff mit der äußeren Zuführröhre (9) verbunden ist;
wobei der Zugstab und das Schubelement mit Ausstoßmechanismen versehen sind und der Zugstab und das Schubelement mittels einer Feder zusammenwirkend angetrieben werden; und wobei die äußere Zuführröhre außerhalb der Zuführnadel montiert ist, sich die Schieberöhre innerhalb der Zuführnadel befindet und die Schieberöhre dazu konfiguriert ist, die Repositionskomponente (21) innerhalb der Zuführnadel freizugeben;
wobei die Repositionskomponente einen Anker und einen Verbindungsdraht umfasst;
**dadurch gekennzeichnet, dass**
die Zuführvorrichtung ferner eine Positionierungsröhre (19) umfasst,
wobei die Positionierungsröhre außerhalb der äußeren Zuführröhre montiert ist und die äußere Röhrenklemme dazu konfiguriert ist, die Positionierungsröhre und die äußere Zuführröhre zu klemmen, sodass ein distales Ende der Positionierungsröhre parallel zu einem distalen Ende der äußeren Zuführröhre ist.

2. System zum Reparieren der Klappensegel durch minimalinvasive Chirurgie nach Anspruch 1, wobei der Zugstab (1) mit einem Evakuierungsverbinder (17) und einer röhrenförmigen Struktur (4), die fähig ist, Blutung zu stillen, und abgeschnitten zu werden, um den Verbindungsdraht zu trennen, versehen ist und ein Federmechanismus zwischen einer Außenseite des Zugstabs und einer Innenseite des Schubelements bereitgestellt ist.

3. System zum Reparieren der Klappensegel durch minimalinvasive Chirurgie nach Anspruch 1, wobei ein Schnappelement (5) auf der Innenseite des Schubelements (2) bereitgestellt ist und dazu konfiguriert ist, ein Ausstoßen des Zugstabs (1) zu kontrollieren, und ein Federmechanismus zwischen einer Außenseite des Schubelements und einer Innenseite des Griffs (3) bereitgestellt ist.

4. System zum Reparieren der Klappensegel durch minimalinvasive Chirurgie nach Anspruch 3, wobei das Gleitelement (6) an dem Griff bereitgestellt ist, wobei sich ein Freigabeauslöserteil in dem Schubelement befindet, wobei der Ziehgriff (2) dazu angeordnet ist, zu ermöglichen, dass das Gleitelement als Reaktion darauf, dass sich der Ziehgriff in einer Richtung weg von dem Griff (3) bewegt, in eine Freigabestellung gleitet, um das Schnappelement zu entsperren und den Zugstab (1) freizugeben.

5. System zum Reparieren der Klappensegel durch minimalinvasive Chirurgie nach Anspruch 3, wobei die Freigabetaste (7) an dem Griff bereitgestellt ist, das Schubelement ausgestoßen wird, nachdem die Freigabetaste ausgelöst wird, und das Gleitelement (6) das Schnappelement (5) auslösen kann, nachdem das Schubelement ausgestoßen ist, wobei das Schnappelement dazu konfiguriert ist, den Ausstoßmechanismus des Zugstabs (1) als Reaktion darauf, entsperrt zu werden, zu entsperren.

6. System zum Reparieren der Klappensegel durch minimalinvasive Chirurgie nach Anspruch 1, wobei der Griff (3) mit einer Sicherheitstaste (8) zum Verhindern von Fehlbedienungen versehen ist und die Sicherheitstaste dazu konfiguriert ist, zu verhindern, dass die Fehlbedienungen die Freigabetaste (7) auslösen.

7. System zum Reparieren der Klappensegel durch minimalinvasive Chirurgie nach einem der Ansprüche 1-6, wobei der Anker aus einem Metall- oder Polymermaterial hergestellt ist und der Anker an dem Klappensegel verankert ist.

8. System zum Reparieren der Klappensegel durch minimalinvasive Chirurgie nach einem der Ansprüche 1-6, wobei der Verbindungsdraht mit dem Anker verbunden ist und der Verbindungsdraht aus einem Polymermaterial hergestellt ist.

9. System zum Reparieren der Klappensegel durch minimalinvasive Chirurgie nach Anspruch 1, wobei die Repositionskomponente (21) mit der Zuführkomponente verbunden ist; wobei der Bediengriff mit einer dreischichtigen inneren Struktur versehen ist, die, von innen nach außen, die Schieberöhre (11), die Zuführnadel (10) und die äußere Zuführröhre (9) umfasst;
wobei der Bediengriff mit einer ersten Röhrenaufnahme (14), einer zweiten Röhrenaufnahme (15) und einer dritten Röhrenaufnahme (16) hintereinander in einer Erstreckungsrichtung weg von dem Evakuierungsverbinder und zu der äußeren Zuführröhre hin versehen ist; wobei der Zugstab mit der ersten Röhrenaufnahme verbunden ist, das Schubelement mit der zweiten Röhrenaufnahme verbunden ist und der Griff mit der dritten Röhrenaufnahme verbunden ist;
wobei ein Ende der Schieberöhre mit der ersten Röhrenaufnahme verbunden ist und das andere Ende der Schieberöhre durch die Mitte der zweiten Röhrenaufnahme läuft;
wobei ein Ende der Zuführnadel mit der zweiten Röhrenaufnahme verbunden ist und das andere Ende der Zuführnadel durch die Mitte der dritten Röhrenaufnahme läuft;
wobei ein Ende der äußeren Zuführröhre mit der dritten Röhrenaufnahme verbunden ist; wobei das andere Ende der äußeren Zuführröhre durch die Mitten der äußeren Röhrenklemme und der Positionierungsröhre läuft; und
der Anker mit dem Verbindungsdraht verbunden ist.

10. System zum Reparieren der Klappensegel durch minimalinvasive Chirurgie nach Anspruch 9, wobei die erste Röhrenaufnahme (14) mit einer ersten Eingriffsnut (22) versehen ist, die Schieberöhre (11) mit einem ersten Eingriffsstück (25) versehen ist und die erste Eingriffsnut (22) mit dem ersten Eingriffsstück (25) zusammenpasst;
wobei die zweite Röhrenaufnahme (15) mit einer zweiten Eingriffsnut (23) versehen ist, die Zuführnadel (10) mit einem zweiten Eingriffsstück (26) versehen ist und die zweite Eingriffsnut mit dem zweiten Eingriffsstück zusammenpasst, und
wobei die dritte Röhrenaufnahme (16) mit einer dritten Eingriffsnut versehen ist, die äußere Zuführröhre mit einem dritten Eingriffsstück (27) versehen ist und die dritte Eingriffsnut mit dem dritten Eingriffsstück zusammenpasst.

11. System zum Reparieren der Klappensegel durch minimalinvasive Chirurgie nach Anspruch 9, wobei ein erster Federmechanismus zwischen einer Außenseite des Zugstabs (1) und einer Innenseite des Schubelements (2) bereitgestellt ist und der Zugstab mittels des ersten Federmechanismus antreibend mit dem Schubelement verbunden ist; und
ein zweiter Federmechanismus zwischen einer Außenseite des Schubelements und einer Innenseite des Griffs (3) bereitgestellt ist und das Schubelement mittels des zweiten Federmechanismus antreibend mit dem Griff verbunden ist.

12. System zum Reparieren der Klappensegel durch minimalinvasive Chirurgie nach Anspruch 9, wobei der Zugstab (1) mit einem Evakuierungsverbinder (17), einer Dehnvorrichtung, einer röhrenförmigen Struktur (4) und einem zum Gleiten in dem Schubelement (2) angeordneten ersten Gleitstück (18) versehen ist und die röhrenförmige Struktur geformt ist, um Bluten zu stillen und betätigbar ist, um von dem Verbindungsdraht getrennt zu werden, wodurch der Anker von der Zuführvorrichtung gelöst wird; und
wobei die Dehnvorrichtung einen Drahtclip (28) und eine erste Feder umfasst und der Drahtclip mit dem Verbindungsdraht verbunden ist;
und wobei optional
das Schubelement inwendig mit einer ersten Gleitnut (30) versehen ist, die es dem Zugstab ermöglicht, zu gleiten, und die erste Gleitnut an das erste Gleitstück angepasst ist; und ein zweites Gleitstück (32), das dazu angeordnet ist, in dem Griff (3) zu gleiten, außerhalb des Schubelements bereitgestellt ist;
und wobei ferner optional
das Schubelement mit einer quadratischen Struktur versehen ist, ein Schnappelement (5) in der quadratischen Struktur bereitgestellt ist, ein Ende des Schnappelements mit einer zweiten Feder versehen ist und das Schnappelement betätigbar ist, um den Zugstab unter der Kontrolle der zweiten Feder zu sperren;
und wobei noch ferner optional
der Griff mit einem Gleitelement und einer zweiten Gleitnut (31), die es dem Schubelement ermöglicht, zu gleiten, versehen ist und die zweite Gleitnut an das zweite Gleitstück angepasst ist; und wobei sich das Gleitelement an einem hinteren Teil des Griffs befindet, wobei das Gleitelement dazu angeordnet ist, in eine Freigabestellung zu gleiten, nachdem das Schubelement herausgezogen wird, und das Gleitelement betätigbar ist, um das Schnappelement zu entsperren, wenn das Schubelement ausgestoßen wird, wobei das Schnappelement dazu konfiguriert ist, als Reaktion darauf, entsperrt zu werden, den Ausstoßmechanismus des Zugstabs zu entsperren.

13. System zum Reparieren der Klappensegel durch minimalinvasive Chirurgie nach Anspruch 9, wobei eine Freigabetaste (7) an einem vorderen Ende des Griffs (3) bereitgestellt ist, eine dritte Feder an einem unteren Ende der Freigabetaste bereitgestellt ist und wobei, nachdem das Schubelement (2) herausgezogen wird, die Freigabetaste dazu konfiguriert ist, das Schubelement unter der Wirkung der dritten Feder zu sperren und das Schubelement ausgestoßen wird, nachdem die Freigabetaste ausgelöst wird;
und wobei optional
ferner eine Sicherheitstaste (8) an dem Griff bereitgestellt ist, die Sicherheitstaste der Freigabetaste (7) benachbart bereitgestellt ist, eine vierte Feder an einem hinteren Teil der Sicherheitstaste bereitgestellt ist und die Sicherheitstaste dazu konfiguriert ist, mittels einer Kraft der vierten Feder gesperrt zu bleiben;
und wobei ferner optional
ein Sicherheitstasten-Rücksetzloch (33) ferner an dem Griff bereitgestellt ist wobei das Sicherheitstasten-Rücksetzloch der Sicherheitstaste benachbart bereitgestellt ist und das Sicherheitstasten-Rücksetzloch betätigbar ist, um die Sicherheitstaste zurückzusetzen, nachdem die Sicherheitstaste entsperrt ist.

## Revendications

1. Système destiné à réparer des feuillets valvulaires dans une chirurgie à effraction minimale, comprenant :
un dispositif de délivrance et un composant de restauration (21), dans lequel le dispositif de délivrance est configuré pour réaliser une opération de libération du composant de restauration ;
le dispositif de délivrance comprend une poignée de fonctionnement, une pince à tube externe (20), un tube de délivrance externe (9), une aiguille de délivrance (10) et un tube poussoir (11) ; dans lequel la poignée de fonctionnement comprend une tige de traction (1), un élément poussoir (2), une poignée (3), un bouton de libération (7), un élément coulissant (6) et un bouton de sécurité (8) ;
dans lequel la tige de traction est connectée au tube poussoir (11), et l'élément poussoir est ajusté à l'extérieur de la tige de traction ; l'élément poussoir est connecté à l'aiguille de délivrance (10), et la poignée est montée à l'extérieur de l'élément poussoir ; et
dans lequel la poignée est connectée au tube de délivrance externe (9) ;
la tige de traction et l'élément poussoir sont pourvus de mécanismes d'éjection, et la tige de traction et l'élément poussoir sont entraînés en coopération au moyen d'un ressort ; et le tube de délivrance externe est monté à l'extérieur de l'aiguille de délivrance, le tube poussoir est situé à l'intérieur de l'aiguille de délivrance, et le tube poussoir est configuré pour libérer le composant de restauration (21) à l'intérieur de l'aiguille de délivrance ;
dans lequel le composant de restauration comprend une ancre et un fil de connexion ;
**caractérisé en ce que** le dispositif de délivrance comprend en outre un tube de positionnement (19),
dans lequel le tube de positionnement est monté à l'extérieur du tube de délivrance externe et la pince de tube externe est configurée pour serrer le tube de positionnement et le tube de délivrance externe, de sorte qu'une extrémité distale du tube de positionnement est parallèle à une extrémité distale du tube de délivrance externe.

2. Système destiné à réparer les feuillets valvulaires dans une chirurgie à effraction minimale selon la revendication 1, dans lequel la tige de traction (1) est pourvue d'un connecteur d'évacuation (17) et d'une structure tubulaire (4) capable d'arrêter le saignement et d'être coupée pour déconnecter le fil de connexion, et un mécanisme de ressort est fourni entre un côté externe de la tige de traction et un côté interne de l'élément poussoir.

3. Système destiné à réparer les feuillets valvulaires dans une chirurgie à effraction minimale selon la revendication 1, dans lequel un élément d'encliquetage (5) est fourni sur le côté interne de l'élément poussoir (2), et est configuré pour commander une éjection de la tige de traction (1), et un mécanisme de ressort est fourni entre un côté externe de l'élément poussoir et un côté interne de la poignée (3).

4. Système destiné à réparer les feuillets valvulaires dans une chirurgie à effraction minimale selon la revendication 3, dans lequel l'élément coulissant (6) est fourni sur la poignée, une partie de déclenchement de libération est située dans l'élément poussoir, dans lequel la poignée de traction (2) est agencée pour permettre à l'élément coulissant de coulisser vers une position de libération en réponse au mouvement de la poignée de traction dans une direction éloignée de la poignée (3), de manière à déverrouiller l'élément d'encliquetage et libérer la tige de traction (1).

5. Système destiné à réparer les feuillets valvulaires dans une chirurgie à effraction minimale selon la revendication 3, dans lequel le bouton de libération (7) est fourni sur la poignée, l'élément poussoir est éjecté après le déclenchement du bouton de libération, et l'élément coulissant (6) peut déclencher l'élément d'encliquetage (5) après l'éjection de l'élément poussoir, l'élément d'encliquetage configuré pour déverrouiller le mécanisme d'éjection de la tige de traction (1) en réponse à son déverrouillage.

6. Système destiné à réparer les feuillets valvulaires dans une chirurgie à effraction minimale selon la revendication 1, dans lequel la poignée (3) est pourvue d'un bouton de sécurité (8) destiné à empêcher les fonctionnements incorrects, et le bouton de sécurité est configuré pour empêcher les fonctionnements incorrects de déclencher le bouton de libération (7).

7. Système destiné à réparer les feuillets valvulaires dans une chirurgie à effraction minimale selon l'une quelconque des revendications 1 à 6, dans lequel l'ancre est faite d'un métal ou d'un matériau polymère, et l'ancre est ancrée dans le feuillet valvulaire.

8. Système destiné à réparer les feuillets valvulaires dans une chirurgie à effraction minimale selon l'une quelconque des revendications 1 à 6, dans lequel le fil de connexion est connecté à l'ancre, et le fil de connexion est fait d'un matériau polymère.

9. Système destiné à réparer les feuillets valvulaires dans une chirurgie à effraction minimale selon la revendication 1, dans lequel le composant de restauration (21) est connecté au dispositif de délivrance ; la poignée de fonctionnement est pourvue d'une structure interne à trois couches, comprenant le tube poussoir (11), l'aiguille de délivrance (10) et le tube de délivrance externe (9) de l'intérieur vers l'extérieur ;
la poignée de fonctionnement est pourvue d'un premier siège de tube (14), d'un deuxième siège de tube (15) et d'un troisième siège de tube (16) dans l'ordre dans une direction d'extension à l'écart du connecteur d'évacuation et vers le tube de délivrance externe ; la tige de traction est connectée au premier siège de tube, l'élément poussoir est connecté au deuxième siège de tube, et la poignée est connectée au troisième siège de tube ;
une extrémité du tube poussoir est connectée au premier siège de tube, et l'autre extrémité du tube poussoir passe à travers le centre du deuxième siège de tube ;
une extrémité de l'aiguille de délivrance est connectée au deuxième siège de tube, et l'autre extrémité de l'aiguille de délivrance passe à travers le centre du troisième siège de tube ;
une extrémité du tube de délivrance externe est connectée au troisième siège de tube ; l'autre extrémité du tube de délivrance externe passe à travers les centres de la pince de tube externe et du tube de positionnement ; et
l'ancre est connectée au fil de connexion.

10. Système destiné à réparer les feuillets valvulaires dans une chirurgie à effraction minimale selon la revendication 9, dans lequel le premier siège de tube (14) est pourvu d'une première rainure de mise en prise (22), le tube poussoir (11) est pourvu d'une première pièce de mise en prise (25), et la première rainure de mise en prise (22) s'ajuste avec la première pièce de mise en prise (25) ;
le deuxième siège de tube (15) est pourvu d'une deuxième rainure de mise en prise (23), l'aiguille de délivrance (10) est pourvue d'une deuxième pièce de mise en prise (26), et la deuxième rainure de mise en prise s'ajuste avec la deuxième pièce de mise en prise ; et
le troisième siège de tube (16) est pourvu d'une troisième rainure de mise en prise, le tube de délivrance externe est pourvu d'une troisième pièce de mise en prise (27), et la troisième rainure de mise en prise s'ajuste avec la troisième pièce de mise en prise.

11. Système destiné à réparer les feuillets valvulaires dans une chirurgie à effraction minimale selon la revendication 9, dans lequel un premier mécanisme de ressort est fourni entre un côté externe de la tige de traction (1) et un côté interne de l'élément poussoir (2), et la tige de traction est connectée par entraînement à l'élément poussoir au moyen du premier mécanisme de ressort ; et
un deuxième mécanisme de ressort est fourni entre un côté externe de l'élément poussoir et un côté interne de la poignée (3), et l'élément poussoir est connecté par entraînement à la poignée au moyen du deuxième mécanisme de ressort.

12. Système destiné à réparer les feuillets valvulaires dans une chirurgie à effraction minimale selon la revendication 9, dans lequel la tige de traction (1) est pourvue d'un connecteur d'évacuation (17), d'un dispositif d'étirement, d'une structure tubulaire (4), et d'un premier coulisseau (18) agencé pour coulisser dans l'élément poussoir (2), et la structure tubulaire façonnée pour arrêter le saignement et capable de fonctionner pour être déconnectée du fil de connexion, dégageant ainsi l'ancre du dispositif de délivrance ; et
le dispositif d'étirement comprend un attache-fil (28) et un premier ressort, et l'attache-fil est connecté au fil de connexion ;
et facultativement,
dans lequel l'élément poussoir est pourvu de manière interne d'une première rainure de coulissement (30) permettant à la tige de traction de coulisser, et la première rainure de coulissement est adaptée au premier coulisseau ; et un deuxième coulisseau (32) agencé pour coulisser au sein de la poignée (3) est fourni à l'extérieur de l'élément poussoir ;
et facultativement en outre,
dans lequel l'élément poussoir est pourvu d'une structure carrée, un élément d'encliquetage (5) est fourni dans la structure carrée, une extrémité de l'élément d'encliquetage est pourvue d'un deuxième ressort, et l'élément d'encliquetage est capable de fonctionner pour verrouiller la tige de traction sous la commande du deuxième ressort ;
et facultativement encore en outre,
dans lequel la poignée est pourvue d'un élément coulissant et d'une deuxième rainure de coulissement (31) permettant à l'élément poussoir de coulisser, et la deuxième rainure de coulissement est adaptée au deuxième coulisseau ; et l'élément coulissant est situé au niveau d'une partie queue de la poignée, l'élément coulissant agencé pour coulisser vers une position de libération après le retrait de l'élément poussoir, et l'élément coulissant est capable de fonctionner pour déverrouiller l'élément d'encliquetage quand l'élément poussoir est éjecté, l'élément d'encliquetage configuré pour déverrouiller le mécanisme d'éjection de la tige de traction en réponse à son déverrouillage.

13. Système destiné à réparer les feuillets valvulaires dans une chirurgie à effraction minimale selon la revendication 9, dans lequel un bouton de libération (7) est fourni au niveau d'une extrémité avant de la poignée (3), un troisième ressort est fourni au niveau d'une extrémité inférieure du bouton de libération, après le retrait de l'élément poussoir (2), le bouton de libération est configuré pour verrouiller l'élément poussoir sous l'action du troisième ressort, et l'élément poussoir est éjecté après le déclenchement du bouton de libération ;
et facultativement,
dans lequel un bouton de sécurité (8) est fourni en outre sur la poignée, le bouton de sécurité est fourni adjacent au bouton de libération (7), un quatrième ressort est fourni au niveau d'une partie queue du bouton de sécurité, et le bouton de sécurité est configuré pour rester verrouillé au moyen d'une force du quatrième ressort ;
et facultativement en outre,
dans lequel un trou de réinitialisation de bouton de sécurité (33) est fourni en outre sur la poignée, le trou de réinitialisation de bouton de sécurité est fourni adjacent au bouton de sécurité, et le trou de réinitialisation de bouton de sécurité est capable de fonctionner pour réinitialiser le bouton de sécurité après le déverrouillage du bouton de sécurité.
